# EUROPEAN PATENT SPECIFICATION

(11) **EP 3 279 309 B1**
(45) Date of publication and mention of the grant of the patent: **21.10.2020**
(21) Application number: 16182655.7
(22) Date of filing: 03.08.2016
(51) Int. Cl.: C12M 1/33

(54) **PLANT FOR TREATING BIOMASS**
ANLAGE ZUR AUFBEREITUNG VON BIOMASSE
INSTALLATION DE TRAITEMENT DE LA BIOMASSE

(43) Date of publication of application: 07.02.2018
(73) Proprietor: Three ES S.r.l., 20824 Lazzate (MB) (IT)
(72) Inventor: SOLDO, Marco, 20824 Lazzate (IT)
(74) Representative: Perani & Partners S.p.A.

(56) References cited:
- EP-A1- 1 884 564
- US-A1- 2008 281 131

## Description

The present invention relates to a system for biomass treatment by means of a controlled hydrodynamic cavitation process. Particularly, the system of the present invention is designed for treatment of wet biomass. This system advantageously finds use in the field of renewable energy, e.g. in the preparation of biomass to be fed to biogas, biomethane, bioethanol power plants. The treated biomass may include agricultural, industrial or residential biomass, such as corn silage, cut grass, straw, organic solid urban waste, grape stalks and grape stalk waste and else.

A system for biomass pretreatment which comprises solid parts mixed with a liquid phase, by cavitation, is known in the art. For example, EP1884564 A1 discloses a device for processing of liquid manure and organic solids which comprises an homogenizer which is configured to treat the biomass by a shera and cavitation mechanisms comprising a plurality of shear blades mutually separated apart along a circunferential direction. US2008281131 discloses a system for alcohol production where grain-based liquid is passed through a controlled flow cavitation apparatus. Cavitation is produced by forcing a flow of grain-based liquid at specific pressure through an orifice, thus breaking the grains and reducing particle size.

Cavitators are disclosed in e.g. EP 610 914 and EP 1 289 638.

As it comes out of the cavitator, the biomass is in a "pumpable" phase, i.e. predominantly liquid. Then such biomass may be used for the above mentioned processes.

### SUMMARY OF THE INVENTION

Nevertheless, such cavitator is not able to operate if it is filled with solid-phase biomass, as water in the biomass is in the form of moisture held within the fiber and is not available for the cavitation process.

Therefore, the technical purpose of the present invention is to provide a system for biomass treatment that ensures a greater efficiency.

A further technical purpose of the present invention is to make use of the controlled hydrodynamic cavitation process using the liquid in the biomass, without adding any liquid except that required for activating the process, to convert the biomass from solid to liquid and hence from shovelable to pumpable.

The aforementioned technical purpose and objects are substantially fulfilled by a system for biomass treatment that comprises the technical features as disclosed in one or more of the accompanying claims.

Particularly, a first embodiment of the invention relates to a system for biomass treatment of the present invention which comprises a biomass disintegration, extraction and homogenization unit. Such disintegration, extraction and homogenization unit is configured to convert biomass from a solid phase to a liquid phase. The disintegration, extraction and homogenization unit has an inlet port and an outlet port.

The system further comprises an outlet conduit connected to the outlet port for conveying the liquid phase of the biomass.

Feed means for the disintegration, extraction and homogenization unit are in fluid communication with the inlet port.

A recirculation line is in fluid communication with the outlet conduit. The feed means comprise a mixer having an inlet port for a solid phase of the biomass. The mixer also has an outlet port in fluid communication with the inlet port of the disintegration, extraction and homogenization unit. The recirculation line is in fluid communication with the mixer to introduce part of the liquid phase of the biomass into the mixer, so that it can be mixed with the solid phase.

Preferably, the disintegration, extraction and homogenization unit comprises a cavitator. Optionally, the system comprises a liquid source that is adapted to be connected for fluid communication with the recirculation line to introduce a fluid into the recirculation line, preferably for starting the circuit only.

A second embodiment relates to a method of biomass treatment, characterized in that it comprises the step of preparing a solid phase of the biomass. The next step is disintegrating such solid phase, and convert it onto a liquid phase through a cavitation process.

The disintegration, extraction and homogenization step is followed by a step of recirculating part of the liquid phase while mixing it with a new portion of solid-phase biomass to obtain a mixture containing some solid phase of the biomass.

After the recirculating step, the disintegration, extraction and homogenization step is repeated to convert the solid phase-biomass contained in the mixture into a liquid phase.

The device and method can solve the technical problem. The above discussed closed-cycle biomass treatment, which is performed by recirculating part of the liquid phase and mixing it with the solid phase, allows an optimized liquid-solid mixture to be introduced into the cavitator, using the liquid contained in the fiber, while still ensuring that cavitation is activated.

### LIST OF DRAWINGS

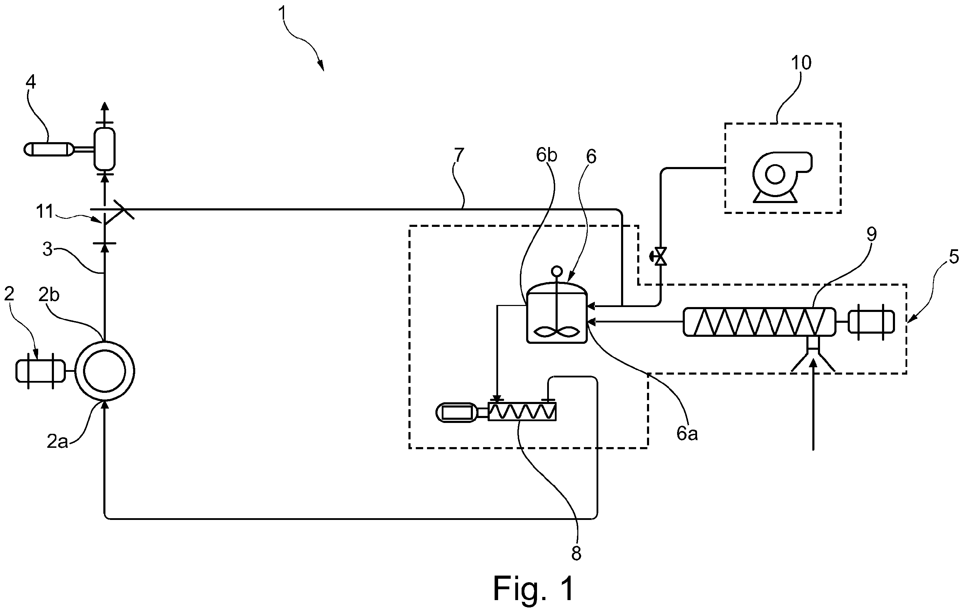

Further features and advantages of the present invention will appear more clearly from the illustrative, non-limiting description of a preferred, non-exclusive embodiment of a system for biomass treatment as shown in the accompanying Figure 1, which shows a schematic view of a system for biomass treatment of the present invention.

### DETAILED DESCRIPTION

Referring to the annexed figures, numeral 1 generally designates a system for biomass treatment of the present invention.

The system 1 comprises a biomass disintegration, extraction and homogenization unit. Such disintegration, extraction and homogenization unit 2 is configured to convert biomass from a solid phase to a liquid phase.

As used herein, the term "solid phase" is intended to designate a material defined by incoherent solid particles, such as corn silage, shredded grass, straw and else. It shall be noted that, since the system is designed for wet biomass treatment, the term "solid phase" does not imply that no liquid component is present, but only relates to the behavior of the material as an incoherent solid mixture.

Likewise, the term "liquid phase" is intended to designate a material that is predominantly in sludge, liquid or semi-liquid phase. This definition does not exclude the presence of suspended solid residues, but only relates to the "pumpable" behavior of the mixture, i.e. the possibility of hydraulically treating the biomass as a liquid and not as an incoherent solid.

The disintegration, extraction and homogenization unit 2 has an inlet port 2a and an outlet port 2b. According to the present invention, the disintegration, extraction and homogenization unit 2 is defined by a cavitation unit. This cavitation unit, not shown, comprises a housing with a rotor accommodated therein. The rotor is mounted to a shaft, which is driven by a motor, preferably an electric motor. The rotor has a series of holes or grooves of different geometries on its outer cylindrical or conical surface, with a cavitation area being generated within the holes or grooves when the rotor is rotated.

The cavitation operation of the disintegration, extraction and homogenization unit 2 is known per se, and will not be described in further detail. In any case, for improved operation of the system 1, the cavitation unit as disclosed in the European Patent Application EP3097973A1, published on 30.11.2016 by the same Applicant hereof may be used.

An outlet conduit 3 is connected to the outlet port 2b, so that the liquid phase of the biomass can be guided out of the disintegration, extraction and homogenization unit 2. A drain pump 4 is connected downstream from the outlet conduit 3, to feed the liquid-phase biomass to any plant downstream from the system 1.

The system 1 further comprise feed means 5 for the disintegration, extraction and homogenization unit 2. Particularly, these feed means 5 are in fluid communication with the inlet port 2a of the disintegration, extraction and homogenization unit 2, and are used to continuously provide solid-phase biomass at a predetermined rate to the disintegration, extraction and homogenization unit 2.

According to the present invention, a recirculation line 7 is in fluid communication with the outlet conduit 3. Advantageously, this recirculation line 7 allows a portion of liquid-phase biomass to be withdrawn from the outlet conduit 3 and be reintroduced into the feed means 5 to be mixed with a solid biomass portion. Then, the resulting mixture is introduced into the disintegration, extraction and homogenization unit 2 for treatment.

Referring to Figure 1, it can be noted that the feed means 5 comprise a mixture 6 having an inlet port 6a for the solid-phase biomass. The mixer 6 also has an outlet port 6b in fluid communication with the inlet port 2a of the disintegration, extraction and homogenization unit 2. Particularly, the recirculation line 7 is in fluid communication with the mixer 6 to introduce part of the liquid phase of the biomass into the mixer 6. The liquid phase is mixed with the solid phase at predetermined ratios, e.g. 1:1, 1:4, to obtain a mixture that comes out of the outlet port 6b of the mixer 6. The above mentioned disintegration, extraction and homogenization unit 2 acts upon the mixture, particularly the solid-phase portion of the biomass contained therein, to convert all the biomass in the mixture to the liquid phase.

Referring to Figure 1, the feed means 5 comprise a feed pump in fluid communication with the outlet port 6b of the mixer 6 and with the inlet port 2a of the disintegration, extraction and homogenization unit 2. This pump 8 may be of any type suitable for the intended purpose and able to act upon pumpable biomasses, for example and without limitation the pump 8 may be a lobe pump or a progressive cavity pump.

Advantageously, the feed pump 8 ensures a constant flow of the mixture to the disintegration, extraction and homogenization unit.

The feed means 5 further comprise a conveyor 9, which is connected to the inlet port 6a of the mixer 6, to introduce solid-phase biomass into the mixer 6 at predetermined rate and flow. Such conveyor 9 may be of any type suitable for the purpose, and able to act upon incoherent solid materials. For instance, the conveyor 9 may be a screw conveyor.

Preferably, the system may comprise a pressurized liquid source 10 that can be connected in fluid communication with the above mentioned recirculation line 7. Thus a liquid, such as water, sewage sludge, leachate or else may be introduced into the recirculation line 7. Advantageously, this is useful during startup of the system 1 when there is no liquid-phase biomass available to be introduced into the mixer 6. When the system operates in a steady state, an on-off valve 12, placed downstream from the source 10, stops the flow of liquid introduced into the system 1 from the outside. In the embodiment of Figure 1, the source 10 is a pump. In alternative embodiments, not shown, it may be any liquid source suitable for the purpose, such as a water-supply system, a sewage sludge or leachate tank or the like. The drain pump 4 is connected downstream from the outlet conduit 3, in this example via a bypass 11, to adjust the amount of biomass withdrawn from the outlet conduit 3, which is directed in part toward a plant downstream from the system 1 and in part toward the recirculation line 7. Therefore, the drain pump 4 is adapted to draw the pumpable biomass from the closed circuit to transfer part of it to the next process (plant, storage o else) and leave the remaining part in the recirculation line 7.

The present invention further relates to a method of biomass treatment. The method includes an initial step of providing the solid-state biomass, preferably in the feed means 5 and more preferably in the conveyor 9.

The solid-state biomass is then mixed with a part of liquid-phase biomass, particularly in the mixer 5. The resulting mixture contains a part of solid-state biomass.

Then the mixture, and particularly the soild-state portion contained therein, is disintegrated, particularly by means of a cavitation process and more particularly in the disintegration, extraction and homogenization unit 2. Then the biomass is converted into a liquid phase. After the disintegration, extraction and homogenization step, part of the liquid phase so obtained is recirculated to be mixed with a solid phase of the biomass, by repeating the mixing step, and obtaining some more mixture of solid-phase and liquid-phase biomass. The disintegration, extraction and homogenization step may be repeated to convert the solid phase-biomass contained in the mixture into a liquid phase.

## Claims

1. A system (1) for wet biomass treatment, comprising a biomass disintegration, extraction and homogenization unit (2), said disintegration, extraction and homogenization unit (2) having an inlet port (2a) and an outlet port (2b); an outlet conduit (3) connected to said outlet port (2b) to convey a liquid phase of said biomass; feed means (5) for said disintegration, extraction and homogenization unit (2), which are in fluid communication with said inlet port (2a); said disintegration, extraction and homogenization unit (2) being defined by a cavitation unit; said cavitation unit comprising a housing, a shaft placed inside said housing, a rotor mounted on the shaft and having an outer surface, a motor connected to said shaft to actuate said shaft; a recirculation line (7) in fluid communication with said outlet conduit (3), said feed means (5) comprising a mixer (6) having an inlet port (6a) for a solid phase of said biomass and an outlet port (6b) in fluid communication with said inlet port (2a) of said disintegration unit (2), said recirculation line (7) being in fluid communication with said mixer (6) to introduce part of said liquid phase of said biomass into said mixer (6) so that it can be mixed with said solid phase; the cavitation unit being configured to use controlled hydrodynamic cavitation and to convert said biomass from said solid phase to said liquid phase,
**characterized in that:**
- said outer surface of the rotor is cylindrical or conical;
- said rotor has a plurality of holes or grooves on said outer surface;
- said rotor is configured to rotate for generating a cavitation area within the holes or grooves.

2. A system (1) as claimed in any of the preceding claims, **characterized in that** said feed means (5) comprise a feed pump (8) in fluid communication with said outlet port (6b) of said mixer (6) and with said inlet port (2a) of said disintegration, extraction and homogenization unit (2).

3. A system (1) as claimed in any of the preceding claims, **characterized in that** said feed means (5) comprise a conveyor (9) connected to said inlet port (6a) of said mixer (6) to introduce solid-phase biomass into said mixer (6).

4. A system (1) as claimed in any of the preceding claims, **characterized in that** it comprises a liquid source (10) which is adapted to be connected in fluid communication with said recirculation line (7) to introduce a fluid into said recirculation line (7), preferably for the initial filling step.

5. A system (1) as claimed in any of the preceding claims, **characterized in that** it comprises a drain pump (4) connected downstream from the outlet conduit (3) for adjusting the amount of biomass withdrawn from said outlet conduit (3) and directed in part toward a plant downstream from said system (1) for biomass treatment and in part toward a recirculation line (7).

6. A method of wet biomass treatment carried out by the system (1) according to any claims 1 to 5, **characterized in that** it comprises the steps of providing a solid phase of said biomass; disintegrating said solid phase and converting it into a liquid phase through a controlled hydrodynamic cavitation process generating, during the rotation of the rotor, a cavitation area within the holes or grooves formed on the cylindrical or conical outer surface of the rotor; after said disintegration, extraction and homogenization step, recirculating part of said liquid phase while mixing it with a new portion of solid-phase biomass to obtain a mixture containing some solid phase of said biomass; after said recirculation step, repeating said disintegration, extraction and homogenization step to convert the solid phase of said biomass contained in said liquid-phase mixture.

## Patentansprüche

1. System (1) zur Behandlung feuchter Biomasse, umfassend eine Biomasse-Zerfalls-, Extraktions- und Homogenisierungseinheit (2), wobei die Zerfalls-, Extraktions- und Homogenisierungseinheit (2) eine Einlassöffnung (2a) und eine Auslassöffnung (2b); eine Auslassleitung (3), die mit der Auslassöffnung (2b) verbunden ist, um die flüssige Phase der Biomasse zu fördern; Zufuhrmittel (5) für die Zerfalls-, Extraktions- und Homogenisierungseinheit (2), die mit der Einlassöffnung (2a) in Fluidverbindung stehen aufweist; die Zerfalls-, Extraktions- und Homogenisierungseinheit (2) durch eine Kavitationseinheit definiert ist; die Kavitationseinheit ein Gehäuse, eine im Gehäuse untergebrachte Welle, einen auf der Welle montierten und eine Außenfläche aufweisenden Rotor, einen mit der Welle zur Betätigung der Welle verbundenen Motor, eine in Fluidverbindung mit der Auslassleitung (3) stehende Umwälzleitung (7) umfasst; die Zufuhrmittel (5) einen Mischer (6) aufweisend eine Einlassöffnung (6a) für eine feste Phase der Biomasse und eine in Fluidverbindung mit der Einlassöffnung (2a) der Zerfallseinheit (2) stehende Auslassöffnung (6b) umfassen; die Umwälzleitung (7) mit dem Mischer (6) in Fluidverbindung steht, um einen Teil der flüssigen Phase der Biomasse in den Mischer (6) einzuleiten, so dass diese mit der festen Phase gemischt werden kann; die Kavitationseinheit dazu eingerichtet ist, die kontrollierte hydrodynamische Kavitation zu verwenden und die Biomasse von der festen Phase in die flüssige Phase umzuwandeln,
**dadurch gekennzeichnet, dass:**
- die Außenfläche des Rotors zylindrisch oder konisch ist;
- der Rotor eine Vielzahl von Löchern oder Rillen auf seiner Außenfläche aufweist;
- der Rotor dazu ausgebildet ist, sich zu drehen, um ein Kavitationsgebiet in den Löchern oder Rillen zu erzeugen.

2. System (1), wie in irgendeinem der vorgehenden Ansprüche beansprucht, **dadurch gekennzeichnet, dass** die Zufuhrmittel (5) eine Zufuhrpumpe (8) umfassen, die mit der Auslassöffnung (6b) des Mischers (6) und mit der Einlassöffnung (2a) der Zerfalls-, Extraktions- und Homogenisierungseinheit (2) in Fluidverbindung stehen.

3. System (1), wie in irgendeinem der vorgehenden Ansprüche beansprucht, **dadurch gekennzeichnet, dass** die Zufuhrmittel (5) einen mit der Einlassöffnung (6a) des Mischers (6) verbundenen Förderer (9) umfassen, um Festphasenbiomasse in den Mischer (6) einzubringen.

4. System (1), wie in irgendeinem der vorgehenden Ansprüche beansprucht, **dadurch gekennzeichnet, dass** es eine Flüssigkeitsquelle (10) umfasst, die dazu ausgelegt ist, in Fluidverbindung mit der Umwälzleitung (7) verbunden zu werden, um ein Fluid in die Umwälzleitung (7) einzubringen, vorzugsweise für den anfänglichen Füllschritt.

5. System (1), wie in irgendeinem der vorgehenden Ansprüche beansprucht, **dadurch gekennzeichnet, dass** es eine Abflusspumpe (4) umfasst, die stromabwärts der Auslassleitung (3) angeschlossen ist, um die Menge an Biomasse zu regeln, die aus der Auslassleitung (3) entnommen wird und zum Teil in Richtung einer Anlage stromabwärts des Systems (1) zur Biomassenbehandlung und zum Teil in Richtung der Umwälzleitung (7) gerichtet wird.

6. Verfahren zur Behandlung feuchter Biomasse, das vom System (1) nach irgendeinem der Ansprüche von 1 bis 5 durchgeführt wird, **dadurch gekennzeichnet, dass** es die Schritte des Bereitstellens einer festen Phase der Biomasse; des Zerfalls der festen Phase und der Umwandlung dieser in eine flüssige Phase durch einen kontrollierten hydrodynamischen Kavitationsprozess umfasst, der während der Rotordrehung ein Kavitationsgebiet in den Löchern oder Rillen erzeugt, die auf der zylindrischen oder konischen Außenfläche des Rotors ausgebildet sind; wobei nach dem Zerfalls-, Extraktions- und Homogenisierungsschritt, ein Teil der flüssigen Phase zurückgeführt wird, während dieser mit einem neuen Teil der Festphasenbiomasse gemischt wird, um eine Mischung zu erhalten, die einiges an fester Phase der Biomasse enthält; nach dem Umwälzschritt, der Zerfalls-, Extraktions- und Homogenisierungsschritt wiederholt wird, um die in der Flüssigphasen-Mischung enthaltene Festphasenbiomasse umzuwandeln.

## Revendications

1. Un système (1) pour le traitement de biomasse humide, comprenant une unité de désintégration, d'extraction et d'homogénéisation de biomasse (2), ladite unité de désintégration, d'extraction et d'homogénéisation (2) ayant un orifice d'entrée (2a) et un orifice de sortie (2b) ; un conduit de sortie (3) raccordé audit orifice de sortie (2b) pour transporter une phase liquide de ladite biomasse ; des moyens d'alimentation (5) pour ladite unité de désintégration, d'extraction et d'homogénéisation (2), qui sont en communication fluidique avec ledit orifice d'entrée (2a) ; ladite unité de désintégration, d'extraction et d'homogénéisation (2) étant définie par une unité de cavitation ; ladite unité de cavitation comprenant un logement, un arbre placé à l'intérieur dudit logement, un rotor monté sur l'arbre et ayant une surface externe, un moteur raccordé audit arbre pour actionner ledit arbre ; une conduite de recirculation (7) en communication fluidique avec ledit conduit de sortie (3), lesdits moyens d'alimentation (5) comprenant un mélangeur (6) ayant un orifice d'entrée (6a) pour une phase solide de ladite biomasse et un orifice de sortie (6b) en communication fluidique avec ledit orifice d'entrée (2a) de ladite unité de désintégration (2), ladite conduite de recirculation (7) étant en communication fluidique avec ledit mélangeur (6) pour introduire une partie de ladite phase liquide de ladite biomasse dans ledit mélangeur (6) de sorte qu'elle peut être mélangée avec ladite phase solide ; l'unité de cavitation étant configurée pour utiliser une cavitation hydrodynamique contrôlée et pour convertir ladite biomasse de ladite phase solide en ladite phase liquide, **caractérisé en ce que :**
- ladite surface externe du rotor est cylindrique ou conique ;
- ledit rotor a une pluralité de trous ou de rainures sur ladite surface externe ;
- ledit rotor est configuré pour tourner afin de générer une zone de cavitation à l'intérieur des trous ou des rainures.

2. Un système (1) tel que revendiqué dans n'importe lesquelles des revendications précédentes, **caractérisé en ce que** lesdits moyens d'alimentation (5) comprennent une pompe d'alimentation (8) en communication fluidique avec ledit orifice de sortie (6b) dudit mélangeur (6) et avec ledit orifice d'entrée (2a) de ladite unité de désintégration, d'extraction et d'homogénéisation (2).

3. Un système (1) tel que revendiqué dans n'importe lesquelles des revendications précédentes, **caractérisé en ce que** lesdits moyens d'alimentation (5) comprennent un convoyeur (9) raccordé audit orifice d'entrée (6a) dudit mélangeur (6) pour introduire de la biomasse en phase solide dans ledit mélangeur (6).

4. Un système (1) tel que revendiqué dans n'importe lesquelles des revendications précédentes, **caractérisé en ce qu'**il comprend une source liquide (10) qui est conçue pour être raccordée en communication fluidique avec ladite conduite de recirculation (7) afin d'introduire un fluide dans ladite conduite de recirculation (7), préférablement pour l'étape de remplissage initiale.

5. Un système (1) tel que revendiqué dans l'une quelconque des revendications précédentes, **caractérisé en ce qu'**il comprend une pompe de vidange (4) raccordée en aval du conduit de sortie (3) pour ajuster la quantité de biomasse retirée dudit conduit de sortie (3) et dirigée en partie vers une installation en aval dudit système (1) pour le traitement de biomasse et en partie vers une conduite de recirculation (7).

6. Un procédé de traitement de biomasse humide mis en œuvre par le système (1) selon n'importe lesquelles des revendications 1 à 5, **caractérisé en ce qu'**il comprend les étapes consistant à fournir une phase solide de ladite biomasse ; désintégrer ladite phase solide et convertir celle-ci en une phase liquide par l'intermédiaire d'un processus de cavitation hydrodynamique contrôlée générant, pendant la rotation du rotor, une zone de cavitation à l'intérieur des trous ou des rainures formé(e)s sur la surface externe cylindrique ou conique du rotor ; après ladite étape de désintégration, d'extraction et d'homogénéisation, faire recirculer une partie de ladite phase liquide tout en la mélangeant avec une nouvelle partie de biomasse en phase solide pour obtenir un mélange contenant de la phase solide de ladite biomasse ; après ladite étape de recirculation, répéter ladite étape de désintégration, d'extraction et d'homogénéisation pour convertir la phase solide de ladite biomasse contenue dans ledit mélange en phase liquide.
